# EUROPEAN PATENT SPECIFICATION

(11) **EP 4 153 555 B1**
(45) Date of publication and mention of the grant of the patent: **31.01.2024**
(21) Application number: 21728192.2
(22) Date of filing: 20.05.2021
(51) Int. Cl.: C07C 46/08, C07C 50/04

(54) **PHOTOOXIDATION OF PHENOLIC COMPOUNDS**
PHOTOOXIDATION VON PHENOLISCHEN VERBINDUNGEN
PHOTO-OXYDATION DE COMPOSÉS PHÉNOLIQUES

(30) Priority: 20.05.2020 EP 20175623
(43) Date of publication of application: 29.03.2023
(73) Proprietor: DSM IP Assets B.V., 6411 TE Heerlen (NL); Universität Basel, 4056 Basel (CH)
(72) Inventor: BONRATH, Werner, 4303 Kaiseraugst (CH); MEDLOCK, Jonathan Alan, 4303 Kaiseraugst (CH); SCHUETZ, Jan, 4303 Kaiseraugst (CH); SPARR, Christof, 4056 Basel (CH); BUCHHOLZ, Thomas, 4056 Basel (CH); MILADINOV, Dragan, 4056 Basel (CH); WELLAUER, Joël, 4056 Basel (CH)
(74) Representative: dsm-firmenich IP
(86) International application number: PCT/EP2021/063448
(87) International publication number: WO 2021/234080

(56) References cited:
- SUCHARD O ET AL: "Photooxygenations of 1-naphthols: an environmentally friendly access to 1,4-naphthoquinones", TETRAHEDRON, ELSEVIER SCIENCE PUBLISHERS, AMSTERDAM, NL, vol. 62, no. 7, 13 February 2006 (2006-02-13), pages 1467-1473, XP025002325, ISSN: 0040-4020, DOI: 10.1016/J.TET.2005.11.021 [retrieved on 2006-02-13]
- ROBERT GERDES ET AL: "Photo-oxidation of phenol and monochlorophenols in oxygen-saturated aqueous solutions by different photosensitizers", JOURNAL OF PHOTOCHEMISTRY AND PHOTOBIOLOGY, A: CHEMISTRY., vol. 111, no. 1-3, 30 June 1997 (1997-06-30), pages 65-74, XP055744501, CH ISSN: 1010-6030, DOI: 10.1016/S1010-6030(97)00209-8
- GOTTHARD WURM ET AL: "Farbstoffsensibilisierte Photooxidation von 1-Naphtholen mit Alkyl-und Alkoholfunktionen in 2-Position", ARCHIV DER PHARMAZIE (WEINHEIM), vol. 319, 1 January 1986 (1986-01-01), pages 97-101, XP055742429,
- OXANA A. KHOLDEEVA ET AL: "Mechanistic Insights into Oxidation of 2-Methyl-1-naphthol with Dioxygen: Autoxidation or a Spin-Forbidden Reaction?", JOURNAL OF PHYSICAL CHEMISTRY PART B, vol. 115, no. 42, 27 October 2011 (2011-10-27), pages 11971-11983, XP055744467, US ISSN: 1520-6106, DOI: 10.1021/jp2055975
- Dina Murtinho ET AL: "Novel porphyrins and a chlorin as efficient singlet oxygen photosensitizers for photooxidation of naphthols or phenols to quinones", Journal of the Chemical Society, Perkin Transactions 2, no. 12, 1 November 2000 (2000-11-01), pages 2441-2447, XP055742436, ISSN: 1470-1820, DOI: 10.1039/b006583h
- 319 Band ET AL: "Archiv der Pharrnazie Farbstoffsensibilisierte Photooxidation von 1-Naphtholen mit Alkyl-und Alkoholfunktionen in 2-Position", Arch. Pharm. (Weinheim), vol. 319, 1 January 1986 (1986-01-01), pages 97-101, XP055744478,

## Description

### Technical Field

The present invention relates to preparation of quinoid compounds and particularly to the field of photooxidation of phenolic compounds.

### Background of the invention

Quinones are an important class of chemicals which can be of broad interest for synthesis of molecules in the field of pharmaceuticals, aroma ingredients, food and feed additives and colorants and dyes.

It has been proposed by Murtinho D. et al., J. Chem. Soc. Perkin Trans. 2, 2000, 2441-2447 to photooxidize naphthalene-1,5-diol to obtain 5-hydroxynaphthalene-1 ,4-dione using oxygen in the presence of a photosensitizer. In particular, it discloses methylene blue as photosensitizer in a mixture of acetonitrile and dichloromethane. However, as a result of moderate yield, it has been suggested using porphyrin type photosensitizers instead. Photooxidation of naphthols is disclosed in: SUCHARD O ET AL: "Photooxygenations of 1-naphthols: an environmentally friendly access to 1,4-naphthoquinones",TETRAHEDRON, ELSEVIER SCIENCE PUBLISHERS, AMSTERDAM, NL, vol. 62, no. 7, 13 February 2006 (2006-02-13), pages 1467-1473, XP025002325,ISSN: 0040-4020, DOI: 10.1016/J.TET.2005.11.021

McQuade D.T. et al. RSC Adv., 2016, 6, 12717-12725 discloses the photooxidation of different 1-naphthol derivates to the respective 1,4-naphtho-quinione derivates using oxygen in the presence of porphyrin type photosensitizers in a mixture of acetonitrile and dichloromethane and white LED as light source in a flow reactor.

However, such porphyrin compounds, as disclosed above, are rather expensive and not readily commercially available. On the other hand acetonitrile as well as dichloromethane are solvents which have significant ecological and ecotoxicological disadvantages.

G. Wurm et al., Arch. Pharm. 319, 97-101 (1986) disclose that 2-alkyl-5-methoxynaphth-1-ols have low yields (<20%) in the respective 1,4-naphtho-quinione derivates when oxidized in a photoreaction. Particularly it discloses that 5-methoxy-2-(2-methylpropyl)-naphth-1-ol in methanol using methylene blue as sensitizer shows a yield less than 3% of the desired 2-isobutyl-5-methoxynaphthalene-1,4-dione.

Methylene blue is often used as photosensitizer in photoreactions and is commercially readily available from various suppliers.

### Summary of the invention

A process for obtaining specific quinones of formula (I) from the respective phenolic compounds is of great interest since phenols are generally widely available and can be produced by known chemical transformations.

It has been surprisingly found that the photooxidation according to claim 1 offers an efficient way of solving this problem.

It has been found that this photooxidation of compound of formula (II) yields the desired product (I) in exceptional high conversions, yield and selectivities.

In the present invention methylene blue can be used, which is a readily available and cost-effective a very attractive photosensitizer and get the desired product not only in very high yield and at high conversion but also in a very high selectivity. Particularly advantageous is that the process can be performed in the absence of any chlorinated solvents. Hence, said process is highly attractive for industrial application.

Further aspects of the invention are subject of further independent claims. Particularly preferred embodiments are subject of dependent claims.

### Detailed description of the invention

The present invention relates to a process of manufacturing the compound of the formula (I) from the compound of the formula (II) by photooxidation 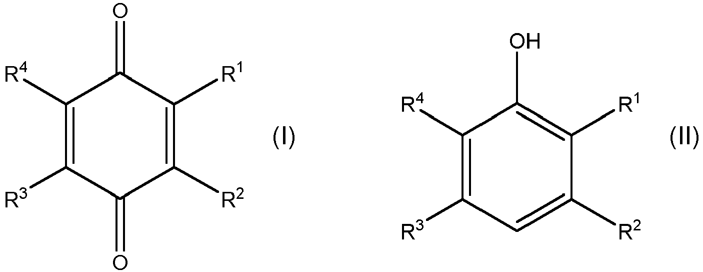
wherein R¹ and R² independently from each other represent either H or a C₁₋₄ alkyl group or a halogenated C₁₋₄ alkyl group, a OR⁹ group or a -CH₂-OR⁹ group;
and wherein R³ and R⁴
   either independently from each other represent H or a C₁₋₄ alkyl group or a halogenated C₁₋₄ alkyl group or a OR⁹ group or a -CH₂-OR⁹ group, particularly H or CH₃;
   or form together a group of the formula 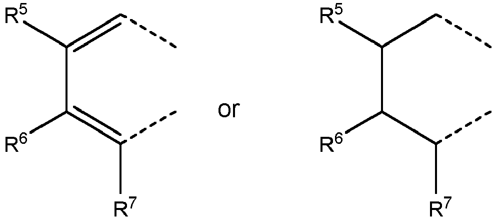
      wherein R⁵ and R⁶ and R⁷ independently from each other represent either H or OH or a C₁₋₄ alkyl group or a OR⁹ group;
      with the proviso that at least two of the residues R⁵ and R⁶ and R⁷ are different from OH;
      the dotted line represents the bond by which the substituent of the formula (is bound to the rest of the compound of formula (I) or formula (II);
wherein R⁹ is a C₁₋₄ alkyl group, particularly CH₃, using oxygen and a photosensitizer of the formula (III) 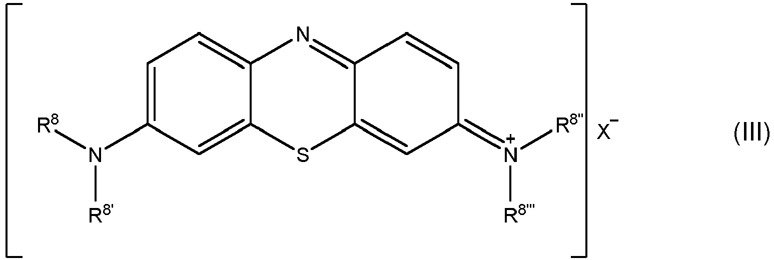
wherein R⁸, R^{8'}, R^{8"} and R^{8‴} independently from each other represent either a H, or a C₁₋₄ alkyl group;
   or
wherein R⁸ and R^{8'} and/or R^{8"} and R^{8‴} form together with N a five or six membered ring;
and X- represents an anion;

with the proviso that
   R⁴ is different from H if R¹=R²=R³ =CH₃ ; and
   R³ is different from H if R¹=R²=R⁴ =CH_{3;} and
   at least one of the residues R¹, R², R³ and R⁴ is different from H and
   at least one of the residues R⁸, R^{8'}, R^{8"} and R^{8‴} is different from H;
in a solvent mixture of water and at least one C₁₋₈ alkanol or at least one C₂₋₄ alkylene diol;
and using light which has a peak wavelength (λₘₐₓ) in its spectrum in the range of between 580 and 780 nm.

For sake of clarity, some terms used in the present document are defined as follows:
In the present document, a "C_{x-y}-alkyl" group is an alkyl group comprising x to y carbon atoms, i.e., for example, a C₁₋₃-alkyl group is an alkyl group comprising 1 to 3 carbon atoms. The alkyl group can be linear or branched. For example -CH(CH₃)-CH₂-CH₃ is considered as a C₄-alkyl group.

Analogously, a C_{x-y} alkanol, respectively a C_{x-y} alkylene diol, is an alcohol having one, respectively two, OH groups where the alcohol has an alkyl respectively alkylene group comprising x to y carbon atoms.

In case identical labels for symbols or groups are present in several formulae, in the present document, the definition of said group or symbol made in the context of one specific formula applies also to other formulae which comprises the same said label.

The peak wavelength is the wavelength where the spectrum reaches its highest intensity.

In the said process compound of formula (II) is photooxidized to yield compound of formula (I). Compound of formula (II) is readily available by syntheses known by the person skilled in the art and/or is commercially available.

In one embodiment, R³ and R⁴ represents independently from each other H or a C₁₋₄ alkyl group or a halogenated C₁₋₄ alkyl group, preferably a CF₃ group, or a OR⁹ group, preferably a OCH₃ group, or a -CH₂-OR⁹ group, preferably a -CH₂-OCH₃ group, particularly H or CH₃

However, in the case that R¹=R²=R³ =CH₃, R⁴ is different from H. Furthermore, in the case that R¹=R²=R⁴ =CH₃, R³ is different from H. Furthermore, at least one of the residues R¹, R², R³ and R⁴ is different from H.

In other words, compound of formula (II) is neither 2,3,6-trimethylphenol nor 2,3,5-trimethylphenol nor phenol.

In this embodiment, it is preferred that at least one, preferably at least two, of the residues R¹, R², R³ and R⁴ is an OR⁹ group, particularly a methoxy group.

Compounds of formula (II) of this embodiment are preferably selected from the group consisting of o-cresol, m-cresol, 2-methoxyphenol, 3- methoxyphenol, 2,3-dimethylphenol, 2,5-dimethylphenol, 2,6-dimethylphenol, 3,5-dimethylphenol, 2-methyl-3-methoxyphenol, 2-methyl-5-methoxyphenol, 2-methoxy-6-methylphenol, 2-methoxy-3-methylphenol, 3-methoxy-5-methylphenol, 2-methoxy-5-methylphenol, 2,3-dimethoxyphenol, 2,5-dimethoxyphenol, 3,5-dimethoxyphenol, 2,6-dimethoxyphenol, 2,3-dimethoxy-6-methylphenol, 2,3-dimethoxy-5-methylphenol, 2,5-dimethoxy-3-methylphenol, 2,6-dimethoxy-3-methylphenol, 3,5-dimethoxy-2-methylphenol, 2-ethoxyphenol, 3-ethoxyphenol, 2,3-diethoxyphenol, 2,5-diethoxyphenol, 2,6-diethoxyphenol, 3,5-diethoxyphenol and 5-isopropyl-2-methylphenol.

In another embodiment, R³ and R⁴ form together a group of the formula
or 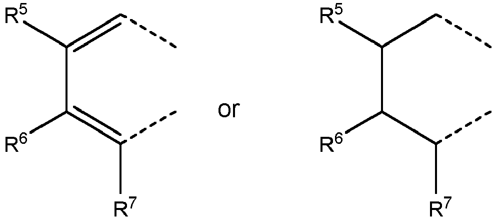 wherein R⁵ and R⁶ and R⁷ independently from each other represent either H or OH or a C₁₋₄ alkyl group or a OR⁹ group;
wherein R⁹ is a C₁₋₄ alkyl group, particularly CH₃, with the proviso that at least two of the residues R⁵ and R⁶ and R⁷ are different from OH;

The compounds of formula (II) of this embodiment are preferably selected from the group consisting of 1-naphthol, 2-methylnaphthalen-1-ol, 3-methylnaphthalen-1-ol, 5-methylnaphthalen-1-ol, 6-methylnaphthalen-1-ol, 7-methylnaphthalen-1-ol, 8-methylnaphthalen-1-ol, 2,3-dimethylnaphthalen-1-ol, 2,5-dimethylnaphthalen-1-ol, 2,6-dimethylnaphthalen-1-ol, 2,7-dimethylnaphthalen-1-ol, 2,8-dimethylnaphthalen-1-ol, 3,5-dimethylnaphthalen-1-ol, 3,6-dimethylnaphthalen-1-ol, 3,7-dimethylnaphthalen-1-ol, 3,8-dimethylnaphthalen-1-ol, 5,6-dimethylnaphthalen-1-ol, 5,7-dimethylnaphthalen-1-ol, 5,8-dimethylnaphthalen-1-ol, 6,7-dimethylnaphthalen-1-ol, 6,8-dimethylnaphthalen-1-ol, 7,8-dimethylnaphthalen-1-ol, 2,3,5-trimethylnaphthalen-1-ol, 2,3,6-trimethylnaphthalen-1-ol, 2,3,7-trimethylnaphthalen-1-ol, 2,3,8-trimethylnaphthalen-1-ol, 5,6,7,8-tetramethylnaphthalen-1-ol, naphthalene-1,5-diol, naphthalene-1,6-diol, naphthalene-1,7-diol, 2-methoxynaphthalen-1-ol, 3-methoxynaphthalen-1-ol, 5-methoxynaphthalen-1-ol, 6-methoxynaphthalen-1-ol and 7-methoxynaphthalen-1-ol, particularly 1-naphthol or 2-methylnaphthalen-1-ol.

It is preferred that R¹ and R² and R³ and R⁴ are independently from each other represent either H or a C₁₋₄ alkyl, preferably H or CH₃, with the proviso that at least one of the substituents R¹ and R² and R³ and R⁴ represents not H.

It is further preferred that R⁵=R⁶=R⁷ =H.

It is further preferred that R²=H and that R¹=H or CH₃.

It is particularly preferred that compound of formula (II) is

In the photooxidation a photosensitizer of the formula (III) is used 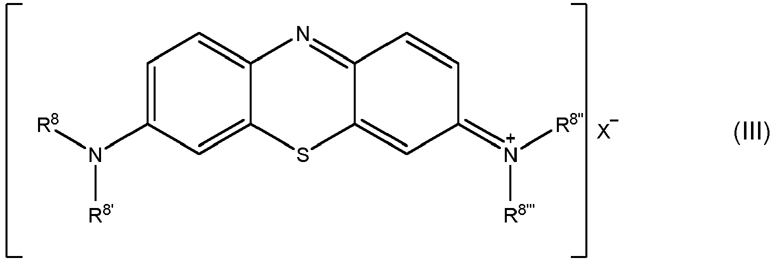
wherein R⁸, R^{8'}, R^{8"} and R^{8‴} independently from each other represent
either a H, or a C₁₋₄ alkyl group;
   or
wherein R⁸ and R^{8'} and/or R^{8"} and R^{8‴} form together with N a five or six
membered ring;
and X- represents an anion;
with the proviso that
at least one of the residues R⁸, R^{8'}, R^{8"} and R^{8‴} is different from H;

In one embodiment, R⁸ and R^{8'} and/or R^{8"} and R^{8‴} form together -(CH₂)₅- or -(CH₂)₂-NH-(CH₂)₂- or -(CH₂)₂-N(C₁₋₄ alkyl)-(CH₂)₂- or -(CH₂)₂-S-(CH₂)₂- or -(CH₂)₂-O-(CH₂)₂- .

It is further preferred that R⁸ = R^{8"} and/or R^{8'} = R^{8‴}. More preferred is that R⁸=R^{8'}=R^{8"}=R^{8‴}.

More preferably, the substituent R⁸, R^{8'}, R^{8"} and R^{8‴} represent a C₁₋₄ alkyl group, even more preferably R⁸=R^{8'}=R^{8"}=R^{8‴} = methyl or ethyl.

Most preferably R⁸=R^{8'}=R^{8"}=R^{8‴}= CH₃.

In formula (III) X⁻ represents an anion. The role of the anion is to counter balance the charge of the cation which is represented in the above formula by the part within the brackets ([)(]). Therefore, in principle any anion can be used.

Preferably, X- represents a halide, most preferably a chloride.

Preferably, the compound of formula (III) is methylene blue. Further preferred is the compound of formula (III) in the form of a double salt with zinc chloride, particularly a double salt of methylene blue with zinc chloride or in the form of a hydrate, preferably methylene blue hydrate (CAS:122965-43-9).

It has been found that the photosensitizer of formula (III) is particularly suited in the photooxidation of the compound of formula (II).

It is essential that for the above photooxidation light is used which has a peak wavelength (λₘₐₓ) in its spectrum in the range of between 580 and 780 nm.

In one preferred embodiment light is used which has a peak wavelength (λₘₐₓ) in its spectrum in the range of between 625 and 740 nm. This corresponds to a light which is perceived as red.

In another, more preferred, embodiment light is used which has a peak wavelength (λₘₐₓ) in its spectrum in the range of between 585 and 625 nm. This corresponds to a light which is perceived as orange.

This light is mainly of the high wavelength range of the visible spectrum.

In a further preferred embodiment the light used is characterized so that more than 80% of the light has a wavelength of between 525 and 780 nm, preferably more than 80% of the light has a wavelength of between 525 and 700 nm, more preferably more than 65% of the emitted light has a wavelength of between 550 and 650 nm.

It is, therefore, important that the light used has no significant amount of light having a wavelength below 580 nm in its spectrum. It is essential that light of the colours green, blue and violet or colours having significant amounts green, blue and violet in their spectrum have been found not to be suited for the above photo oxidation.

In one embodiment, the light which is used for the photooxidation can be realized by filtering the undesired light wavelengths from a light source. For example a light source having a multichromatic or white emission can be filtered by a filter which blocks off the undesired wavelength.

There are different possibility of such filters known and commercially available such as absorption, dichroic, monochromatic, band-pass, short-pass or wedge filters, using different physical methods for filtration of light.

Particularly useful are absorption or cut-off filters.

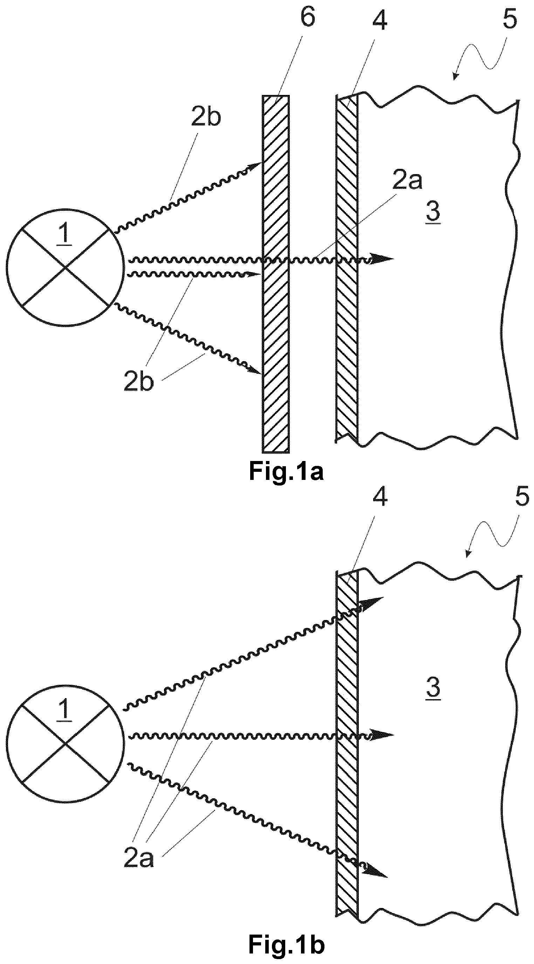

Figure 1a represents a schematic representation of this embodiment. The light source (1) emits radiation of different wavelengths, which have the desired wavelengths (2a) and undesired wavelengths (2b). The light source is preferably a white light, more preferably a white LED. A filter (6) is positioned between light source (1) and the photoreactor which has a transparent wall (4). The filter (6) filters the light of undesired wavelengths to provide a light which has a peak wavelength (λₘₐₓ) in its spectrum in the range of between 580 and 780 nm. The filter (6) is preferably an "orange filter" or "red filter", i.e. a filter that allows only light with a wavelength of between 585 and 625 nm or between 625 and 740 nm to pass through. The reaction mixture (3), which comprises at least oxygen and the compound of formula (II) and the solvent mixture of water and at least one C₁₋₈ alkanol or at least one C₂₋₄ alkylene diol, is inside the photoreactor (5).

By the photoreaction a compound of formula (I) is produced by photochemical reaction from compound of formula (II) and oxygen, particular in a gas mixture comprising at least 20 % by volume of oxygen.

A specific preferred example of this embodiment is a white LED. The light of which is filtered in such a way that all light which is not of the desired wavelengths is blocked or at least significantly absorbed (e.g. using a "orange filter" (allowing transmission of light only of between 585 and 625 nm) or "red filter" (allowing transmission of light only of between 625 and 740 nm)).

So, the light source for the light is preferably a white LED lamp in combination with a filter blocking wavelengths below 500 nm, particularly below 625nm.

In a further embodiment, the light which is used for the photooxidation can be produced by a respective light source emitting light of the desired wavelengths.

Figure 1b represents a schematic representation of this embodiment. The light source (1) emits radiation of the desired wavelengths (2a) to provide a light which has a peak wavelength (λₘₐₓ) in its spectrum in the range of between 580 and 780 nm. The light source is preferably an orange or red light, more preferably an orange or red LED to provide a light using light which has a peak wavelength (λₘₐₓ) in its spectrum in the range of between 580 and 780 nm.

The reaction mixture (3), which comprises at least oxygen and the compound of formula (II) and the solvent mixture of water and at least one C₁₋₈ alkanol or at least one C₂₋₄ alkylene diol, is inside the photoreactor (5). By the photoreaction a compound of formula (I) is produced by photochemical reaction from compound of formula (II) and oxygen.

Specific examples of light sources of this embodiments are red LEDs or red or orange Lasers, preferably red or orange LED lamps. Red and orange LED lamps are commercially broadly available. Red and orange LEDs can provide high intensities of red or orange light. In a preferred embodiments a flexible strip having a plurality of individual LEDs incorporated in said strip. This allows to assure radial orientation of the LEDS around a curved surface such as a transparent tube, for example, by simply wrapping, preferably in a helical manner, said strip around the tube.

The photooxidation is performed in a solvent mixture of water and at least one C₁₋₈ alkanol or at least one C₂₋₄ alkylene diol.

The C₁₋₈ alkanol is preferably selected from the group consisting of methanol, ethanol, propanol, isopropanol, butanol, heptanol and hexanol, more preferably selected from the group consisting of methanol, ethanol and isopropanol.

The C₂₋₄ alkylene diol is preferably selected from the group consisting of ethane-1,2-diol, propane-1,2-diol, propane-1,3-diol, butane-1,3-diol, butane-1,4-diol, butan-1,2-diol and butane -2,3-diol, preferably selected from the group consisting of ethane-1,2-diol, propane-1,2-diol and propane-1,3-diol.

It is preferred that the solvent mixture is a mixture of water and at least one C₁₋₈ alkanol or at least one C₂₋₄ alkylene diol form a homogeneous phase.

It is preferred that the solvent mixture is a mixture of water and at least one C₁₋₈ alkanol or at least one C₂₋₄ alkylene diol. More preferred the solvent mixture is a mixture of water and C₁₋₈ alkanol.

More preferably the solvent mixture is a mixture of water and methanol and/or ethanol and/or isopropanol.

It is preferred that the volume ratio of water to the sum of C₁₋₈ alkanol and C₂₋₄ alkylene diol is in the range of between 1:10 and 1:1, particularly between 1:5 and 1:2

In a very preferred embodiment the solvent mixture is a mixture of water and methanol, preferably in a volume of water to methanol in the range of 1:20 to 1:2, preferably of 1:10 and 1:2, more preferably of 1:6 and 1:3.ratio, most preferably 1:4.

It is a key advantage of the present invention that the photooxidation is made in a solvent mixture consisting of water and at least one C₁₋₈ alkanol or at least one C₂₋₄ alkylene diol, which are ecologically and ecotoxicologically all very favourable solvents and are also economically advantageous. Hence, it is very favourable that the above process is performed in the absence of present of any chlorinated solvent.

It is preferred that the concentration of the compound of formula (II) is in the range of between 0.002 to 2.0 mol/l, preferably 0.01 to 0.2 mol/l at the beginning of the photooxidation.

Further preferred is that the ratio of the compound of formula (III) to the compound of formula (II) is in the range of between 0.005 and 20 mol%, preferably between 0.05 and 20 mol%, more preferably between 0.2 and 10 mol%.

In one embodiment oxygen is used in a form of a mixture comprising oxygen and an inert gas. It is preferred that the amount of oxygen in such a mixture comprising oxygen and an inert gas is at least 15 % by volume, particularly at least 20 % by volume. Such a mixture may, for example, be a binary mixture such as a mixture oxygen/nitrogen or oxygen/argon or alike. Said mixture can consist of or comprise two or more inert gases. It is particularly preferred to use air as such a mixture comprising oxygen and an inert gas.

In a preferred embodiment, oxygen is used in an substantially pure form, i.e. that the amount of oxygen in the gas is 90% -100% , more preferably 95% - 100%, even more preferably 99% - 100%.

The photooxidation can take place at ambient pressure or under pressure. It is preferred that the oxidation takes place under pressure, particularly under a pressure of more than 2 bar, preferably more than 3 bar, more preferably under a pressure of between 2 and 20 bar.

The photooxidation is performed in a suitable photoreactor. A preferred photoreactor is a flow reactor, particularly in a spiral flow reactor.

The individual components can be introduced separately or as mixture into the photo reactor. Preferably the reaction mixture is prepared before entering into the photo reactor.

In one of the preferred embodiments, an oxygen containing solvent mixture is admixed to compound of formula (II) before entering into the photoreactor.

In another preferred embodiment, the solvent mixture is admixed to compound of formula (II) which already contains oxygen before entering into the photoreactor.

In most preferred embodiments, oxygen is added to a pre-mixture which at least comprises compound of formula (II) and the solvent mixture.

The reaction preferably is processed in such a manner that the pressure of oxygen is controlled by suitable valves and mass flow controllers. Such process control equipment and methods for photoreactions using liquids and gases is known by the person skilled in the art.

### Figures

Figure 1a shows a schematic representation of a photooxidation using a light source and a filter to produce a light with a peak wavelength (λₘₐₓ) in its spectrum in the range of between 580 and 780 nm.
Figure 1b shows a schematic representation of a photooxidation using a light source with a peak wavelength (λₘₐₓ) in its spectrum in the range of between 580 and 780 nm.
Figure 2a shows a schematic representation of one of the experimental layout.
Figure 2b shows a schematic representation of a different experimental layout.
Figure 2c shows a schematic representation of another different experimental layout.
Figure 3 represents the normalized emission spectrum of light used for the photooxidation using the orange filter as well as of the white light in the experiments.

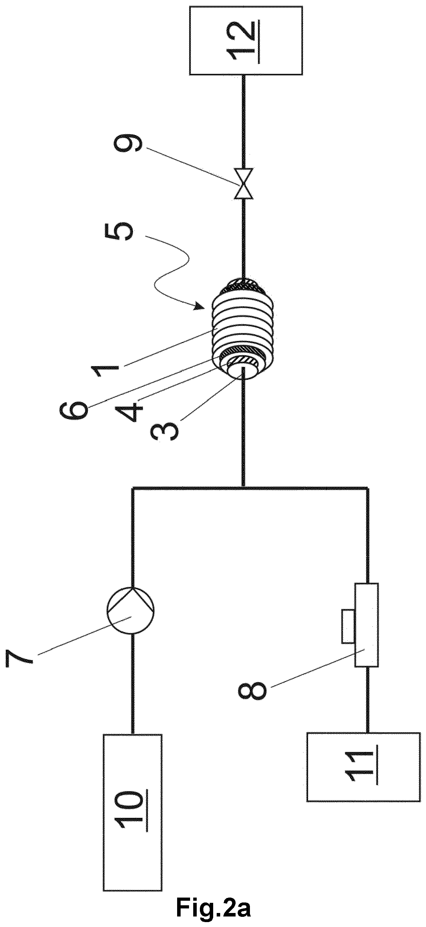

In figure 2a one preferred experimental layout is represented. A vessel comprising a premixture (10), comprising at least the compound of the formula (II) and the photosensitizer of the formula (III) and the solvent mixture of water and at least one C₁₋₈ alkanol or at least one C₂₋₄ alkylene diol, is pumped by a pump (7) into the photoreactor (5). Before entering the photoreactor (5), oxygen (11) is admixed to the premixture forming the photooxidation reaction mixture (3). The amount of oxygen admixed is controlled by a mass flow controller (8). Around the transparent wall (4) of the linear tubular photoreactor (5) the light source (1) is arranged, particularly in a helical arrangement of LEDs. The light source (1) is preferably a white LED. Between the transparent wall (4) and the light source (1) a filter (6) is positioned, allowing to provide a light (2a) which has a peak wavelength (λₘₐₓ) in its spectrum in the range of between 580 and 780 nm. The filter (6) is particularly an orange filter or an red filter, respectively, to provide particularly a light which has a peak wavelength (λₘₐₓ) in its spectrum in the range of between 585 and 625 nm or 625 and 740 nm, respectively. The photoreactor (5) is preferably a spiral flow reactor. At the outlet of the photoreactor a backpressure regulator (9) is positioned before the product is collected in the collection vessel (12).

This experimental layout, particularly the combination of light source and photoreactor, is preferably used for higher volume photoreactions.

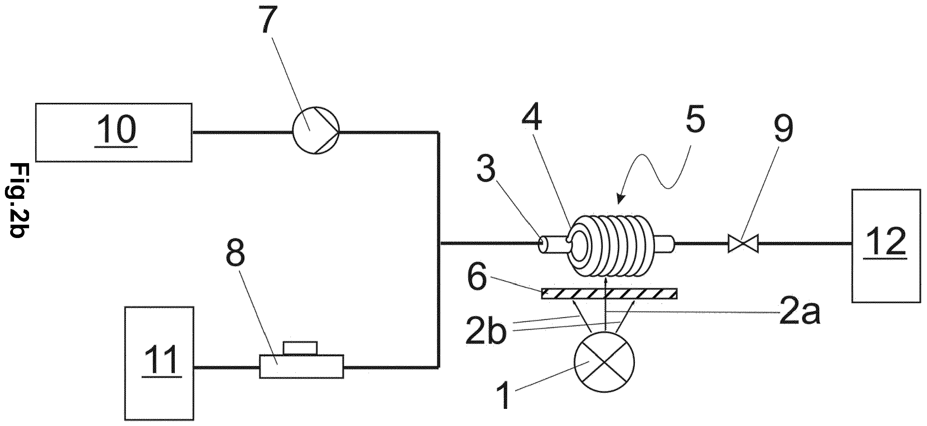

In figure 2b another preferred experimental layout is represented. A vessel comprising a premixture (10), comprising at least the compound of the formula (II) and the photosensitizer of the formula (III) and the solvent mixture of water and at least one C₁₋₈ alkanol or at least one C₂₋₄ alkylene diol, is pumped by a pump (7) into the photoreactor (5). Before entering the photoreactor (5), oxygen (11) is admixed to the premixture forming the photooxidation reaction mixture (3). The amount of oxygen admixed is controlled by a mass flow controller (8).

Between the transparent wall (4) of the photoreactor (5) and the light source (1), preferably white LEDs, a filter (6) is positioned, allowing to provide a light (2a) which has a peak wavelength (λₘₐₓ) in its spectrum in the range of between 580 and 780 nm. In the present representation only one light source (1) and one filter (6) is shown. It is, of course, possible that several such light sources (1), combined with the filter (6) are positioned around the photoreactor (5), in the form of a spiral flow reactor, can be positioned to allow an even irradiation of the whole photoreactor (5). The filter (6) is particularly an orange filter or an red filter, respectively, to provide particularly a light which has a peak wavelength (λₘₐₓ) in its spectrum in the range of between 585 and 625 nm or 625 and 740 nm, respectively. The light having undesired wavelengths (2b) is filtered off by the filter (6). At the outlet of the photoreactor a backpressure regulator (9) is positioned before the product is finally collected in the collection vessel (12).

This experimental layout, particularly the combination of light source and photoreactor, is preferably used for smaller volume photoreactions.

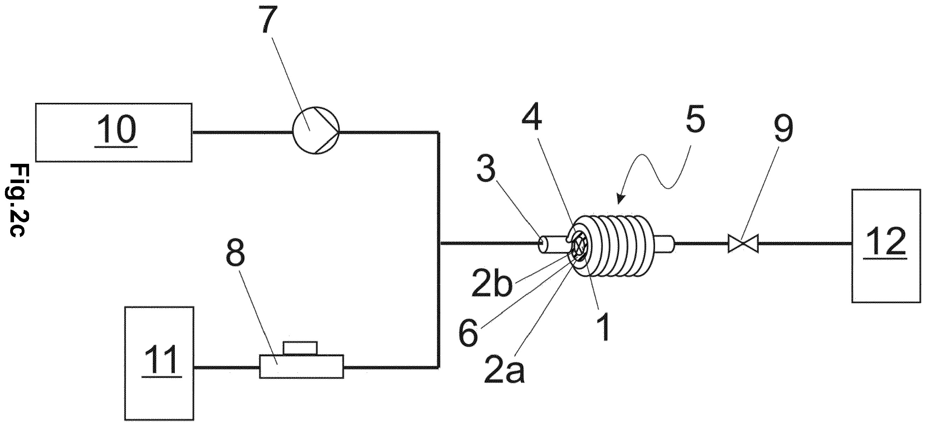

In figure 2c, another preferred experimental layout is represented. A vessel comprising a premixture (10), comprising at least the compound of the formula (II) and the photosensitizer of the formula (III) and the solvent mixture of water and at least one C₁₋₈ alkanol or at least one C₂₋₄ alkylene diol, is pumped by a pump (7) into the photoreactor (5). Before entering the photoreactor (5), oxygen (11) is admixed to the premixture forming the photooxidation reaction mixture (3). The amount of oxygen admixed is controlled by a mass flow controller (8).

In this embodiment a light source (1), preferably white LED, is arranged in the hollow space formed by the helical windings of the spiral flow reactor (5). Around the a light source (1), i.e. between the transparent wall (4) of the photoreactor (5) and the light source (1), a filter (6) is positioned, allowing to provide a light (2a) which has a peak wavelength (λₘₐₓ) in its spectrum in the range of between 580 and 780 nm. The filter (6) is particularly an orange filter or an red filter, respectively, to provide particularly a light which has a peak wavelength (λₘₐₓ) in its spectrum in the range of between 585 and 625 nm or 625 and 740 nm, respectively. The light having undesired wavelengths (2b) is filtered off by the filter (6). At the outlet of the photoreactor a backpressure regulator (9) is positioned before the product is finally collected in the collection vessel (12).

This experimental layout, particularly the combination of light source and photoreactor, is preferably used for smaller volume photoreactions.

In an even further embodiment the light source (1) and filter (6) of figures 2b) and 2c) are combined. In other words the filter and light source can be arranged outside of the photoreactor walls arranged inside as well as outside of the space formed by the helical winding of the spiral flow photoreactor (5).

### List of reference signs

- 1: Light source
- 2a: Light of desired wavelength
- 2b: Light of undesired wavelength
- 3: Photooxidation reaction mixture
- 4: Transparent wall of photoreactor
- 5: Photoreactor
- 6: Filter
- 7: Pump
- 8: Mass flow controller
- 9: Backpressure regulator
- 10: Premixture
- 11: Oxygen
- 12: Collection vessel

### Examples

The present invention is further illustrated by the following experiments.

### Experimental layout

In the following experiments an experimental layout has been used as schematically represented in figure 2c.

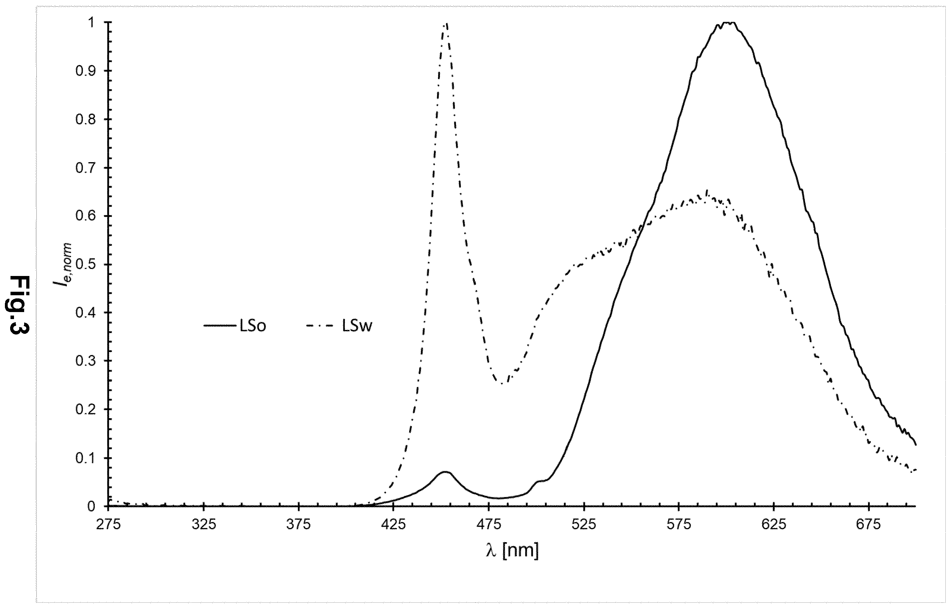

A vessel comprising a premixture (10) of solvent, respectively solvent mixture and the substances to be photooxidized as well as the photosensitizer is pumped by a pump (7) into the photoreactor (5) which is a spiral flow reactor (4.6 mL coil reactor). Before entering the photoreactor (5), oxygen (11) is admixed to the premixture forming the photooxidation reaction mixture (3). The amount of oxygen admixed is controlled by a mass flow controller (8). The light of the light source (1), white LEDs (4000 Im, 32 W, 4100 K)(**LSo**; emission spectrum see figure 3) is filtered by an orange filter (6) to filter off the light of undesired wavelengths (2b) so that the light of the desired wavelengths (2a) is falling on the transparent walls (4) of the photoreactor (5). The photoreactor (5) is coiled over an inner glass cylinder around a LED lamp (1) cooled with a fan and equipped with the respective filter (6) between LED lamp and the wall of the photoreactor (5). At the outlet of the photoreactor a backpressure regulator (9) is positioned before the product is finally collected in the collection vessel (12). The pressure and flow rate of oxygen as well as the residence time (t_{R}) are indicated in the respective experiments.

The amounts of product formed are determined by GC-FID and ¹H-NMR using Durene as internal standard.

The light used for the photooxidation experiments are either
- white LED light (**LSw**)
- white LED light using an orange filter (**LSo**).

In figure 3, the spectra of the light used for the photooxidation experiments between wavelengths of 325 and 700 nm are shown using the normalized relative intensity (*l_{e,norm}*) of the light entering the transparent wall of the photoreactor.

### Experimental series 1

In a first series different substrates have been photooxidized (0.02 mol/L) in a solvent mixture of methanol/water of 4/1 (vol/vol)) using air (10 bar, 1.35 mL/min) and at 35°C and methylene blue (0.9 mol%). (Flowrate 0.25 mL/min, t_{R} = 23 min).The results are compiled in table 1:

**Table 1. Photoreaction of different substrates using air and LSo as light source.**

| | Substrate used | Product | Conversion[%] |
|---|---|---|---|
| ***1*** | 3-methoxy-5-methylphenol | 2-methoxy-6-methyl-benzoquinone | 80 |
| ***2*** | 3,5-dimethylphenol | 2,6-dimethylbenzoquinone | 69 |
| ***3*** | 2,5-dimethylphenol | 2,5-dimethylbenzoquinone | 50 |
| ***4*** | 5-isopropyl-2-methylphenol | 5-isopropyl-2-methylbenzoquinone | 39 |
| ***5*** | 2,6-dimethylphenol | 2,6-dimethylbenzoquinone | 21 |
| ***6*** | 3-methoxyphenol | 2-methoxybenzoquinone | 14 |
| ***Ref.1*** | phenol | benzoquinone | 1 |

### Experimental series 2

In a second series different substrates have been photooxidized (0.02 mol/L in a solvent mixture of methanol/water of 4/1 (vol/vol)) using air (10 bar, 0.25-1.35 mL/min) and at 35°C and methylene blue (0.9 mol%). The residence time has been selected so to achieve a conversion of more than 99%.

The results are compiled in table 2:

**Table 2. Photoreaction of different substrates using air and LSo as light source.**

| | Substrate used | Product | t_{R} [min | Conversion [%] | Product Yield [%] |
|---|---|---|---|---|---|
| ***7*** | 2,5-dimethylPhenol | 2,5-dimethylbenzoquinone | 50 | >99 | 73 |
| ***8*** | 3,5-dimethylPhenol | 3,5-dimethylbenzoquinone | 50 | >99 | 87 |
| ***9*** | 2,6-dimethylphenol | 3,5-dimethylbenzoquinone | 50 | >99 | 17 |
| ***10*** | 2-methyl-5-isopropyl-phenol | 2-isopropyl-5-methylbenzoquinone | 15 | >99 | 35 |
| ***11*** | 3-methoxyphenol | 3-methoxybenzoquinone | 120 | >99 | 91 |
| ***12*** | 3-methoxy-5-methylphenol | 3-m ethoxy-5-m ethyl-benzoquinone | 25 | >99 | 88 |
| ***Ref.2*** | 3-(dimethyl-amino)phenol | 2-(dimethylamino)cyclo-hexa-2,5-diene-1,4-dione | 40 | >99 | 0 |
| ***Ref.3*** | 5-methylbenzene-1,3-diol | 2-hydroxy-6-methylcyclo-hexa-2,5-diene-1,4-dione | 16 | >99 | 0 |

### Experimental series 3

In a third series different substrates have been photooxidized (0.02 mol/L in a solvent mixture of methanol/water of 4/1 (vol/vol)) using air (10 bar, 0.5-1.5 mL/min) and at 35°C and methylene blue (0.9 mol%). The residence time has been selected so to achieve a conversion of more than 99%.

The results are compiled in table 3:

**Table 3. Photoreaction of different substrates using air and LSo as light source.**

| | Substrate used | Product | t_{R} [min | Conversion [%] | Product Yield [%] |
|---|---|---|---|---|---|
| ***13*** | 1-naphthol | naphthalene-1,4-dione | 13 | >99 | 97 |
| ***14*** | 2-methyl-naphthalen-1-ol | 2-methylnaphthalene-1,4-dione | 16 | >99 | 98 |
| ***15*** | naphthalene-1,5-diol | 5-hydroxynaphthalene-1,4-dione | 16 | >99 | 90 |
| ***16*** | naphthalene-1,6-diol | 6-hydroxynaphthalene-1,4-dione | 16 | >99 | 89 |
| ***17*** | naphthalene-1,7-diol | 7-hydroxynaphthalene-1,4-dione | 16 | >99 | 87 |
| ***Ref.4*** | 2-naphthol | naphthalene-2,6-dione | 40 | 0 | 0 |
| ***Ref.5*** | naphthalen-1-yl acetate | naphthalene-1,4-dione | 40 | 0 | 0 |

Tables 1-3 show that the compounds of formula (II) show significantly higher yield into the respective quinone (product) compared to other phenols having similar structure as staring material (substrate) in the photooxidation.

## Claims

1. Process of manufacturing a compound of the formula (I) from a compound of the formula (II) by photooxidation 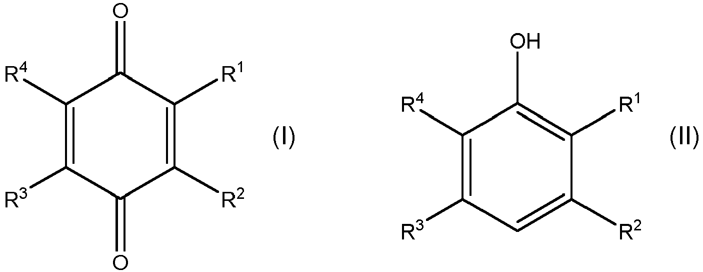
wherein R¹ and R² independently from each other represent either H or a C₁₋₄ alkyl group or a halogenated C₁₋₄ alkyl group, a OR⁹ group or a -CH₂-OR⁹ group;
and wherein R³ and R⁴
either independently from each other represent H or a C₁₋₄ alkyl group or a halogenated C₁₋₄ alkyl group or a OR⁹ group or a -CH₂-OR⁹ group, particularly H or CH₃;
or form together a group of the formula 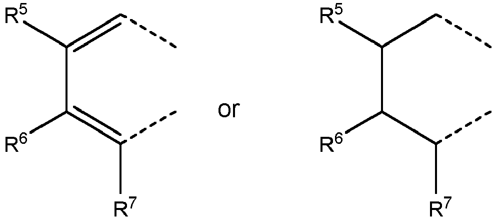
wherein R⁵ and R⁶ and R⁷ independently from each other represent either H or OH or a C₁₋₄ alkyl group or a OR⁹ group;
with the proviso that at least two of the residues R⁵ and R⁶ and R⁷ are different from OH; the dotted line represents the bond by which the substituent of the formula (is bound to the rest of the compound of formula (I) or formula (II);
wherein R⁹ is a C₁₋₄ alkyl group, particularly CH₃,
using oxygen and a photosensitizer of the formula (III) 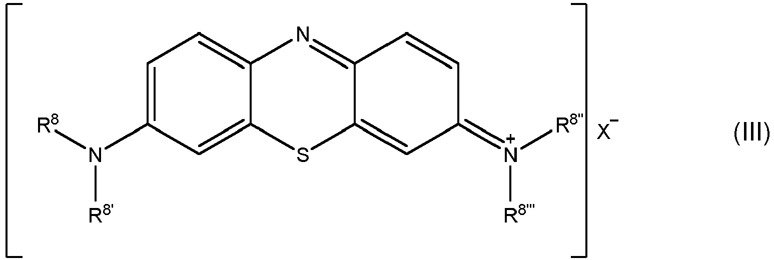
wherein R⁸, R^{8'}, R^{8"} and R^{8‴} independently from each other represent either a H, or a C₁₋₄ alkyl group;
or
wherein R⁸ and R^{8'} and/or R^{8"} and R^{8‴} form together with N a five or six membered ring;
and X- represents an anion;
with the proviso that
R⁴ is different from H if R¹=R²=R³ =CH₃; and
R³ is different from H if R¹=R²=R⁴ =CH₃; and
at least one of the residues R¹, R², R³ and R⁴ is different from H and
at least one of the residues R⁸, R^{8'}, R^{8"} and R^{8‴} is different from H;
in a solvent mixture of water and at least one C₁₋₈ alkanol or at least one C₂₋₄ alkylene diol;
and using light which has a peak wavelength (λₘₐₓ) in its spectrum in the range of between 580 and 780 nm.

2. The process according to claim 1 **characterized in that** used light has a peak wavelength (λₘₐₓ) in its spectrum in the range of between 585 and 625 nm.

3. The process according to claim 1 **characterized in that** used light has a peak wavelength (λₘₐₓ) in its spectrum in the range of between 625 and 740 nm.

4. The process according to any of the preceding claims **characterized in that** more than 80% of the light has a wavelength of between 525 and 780 nm, preferably more than 80% of the emitted light has a wavelength of between 525 and 700 nm, more preferably more than 65% of the light has a wavelength of between 550 and 650 nm.

5. The process according to any of the preceding claims **characterized in that** the solvent mixture is a mixture of water and methanol and/or ethanol and/or isopropanol.

6. The process according to any of the preceding claims **characterized in that** the volume ratio of water to the sum of C₁₋₈ alkanol and C₂₋₄ alkylene diol is in the range of between 1:10 and 1:1, particularly between 1:5 and 1:2.

7. The process according to any of the preceding claims **characterized in that** the light source for the light is a red or orange LED lamp.

8. The process according to any of the preceding claims **characterized in that** the light source for the light is a white LED lamp in combination with a filter blocking wavelengths below 500 nm, particularly below 625 nm.

9. The process according to any of the preceding claims **characterized in that** R⁸=R^{8'}=R^{8"}=R^{8‴}=CH₃.

10. The process according to any of the preceding claims **characterized in that** R¹ and R² and R³ and R⁴ are independently from each other represent either H or a C₁₋₄ alkyl, preferably H or CH₃, with the proviso that at least one of the substituents R¹ and R² and R³ and R⁴ represents not H.

11. The process according to any of the preceding claims 1-9 **characterized in that** R⁵=R⁶=R⁷ =H.

12. The process according to claim 10, **characterized in that** R²=H and that R¹=H or CH₃.

13. The process according to any of the preceding claims **characterized in that** X⁻represents a halide, particularly chloride.

14. The process according to any of the preceding claims **characterized in that** the concentration of the compound of formula (II) is in the range of between 0.002 to 2.0 mol/l, preferably 0.01 to 0.2 mol/l at the beginning of the photooxidation.

15. The process according to any of the preceding claims **characterized in that** the ratio of the compound of formula (III) to the compound of formula (II) is in the range of between 0.005 and 20 mol%, preferably between 0.05 and 20 mol%, more preferably between 0.2 and 10 mol%.

16. The process according to any of the preceding claims **characterized in that** the photooxidation is performed in a flow reactor, particularly in a spiral flow reactor.

## Patentansprüche

1. Verfahren zur Herstellung einer Verbindung der Formel (I) aus einer Verbindung der Formel (II) durch Photooxidation 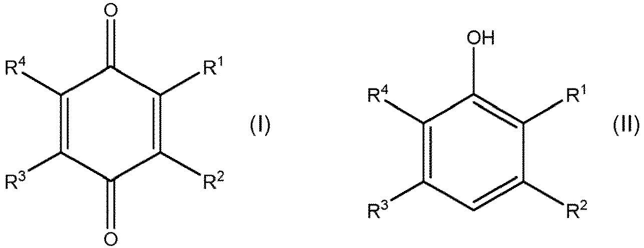
wobei R¹ und R² unabhängig voneinander entweder für H oder eine C₁₋₄-Alkylgruppe oder eine halogenierte C₁₋₄-Alkylgruppe, eine OR⁹-Gruppe oder eine -CH₂-OR⁹-Gruppe stehen;
und wobei R³ und R⁴
entweder unabhängig voneinander für H oder eine C₁₋₄-Alkylgruppe oder eine halogenierte C₁₋₄-Alkylgruppe oder eine OR⁹-Gruppe oder eine -CH₂-OR⁹-Gruppe, insbesondere H oder CH₃, stehen;
oder zusammen eine Gruppe der Formel 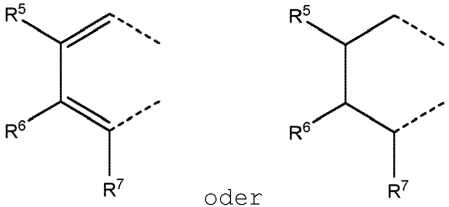
bilden, wobei R⁵ und R⁶ und R⁷ unabhängig voneinander entweder für H oder OH oder eine C₁₋₄-Alkylgruppe oder eine OR⁹-Gruppe stehen;
mit der Maßgabe, dass mindestens zwei der Reste R⁵ und R⁶ und R⁷ von OH verschieden sind;
die gestrichelte Linie für die Bindung steht, über die der Substituent der Formel an den Rest der Verbindung der Formel (I) oder Formel (II) gebunden ist;
wobei R⁹ für eine C₁₋₄-Alkylgruppe, insbesondere CH₃, steht;
unter Verwendung von Sauerstoff und einem Photosensibilisator der Formel (III) 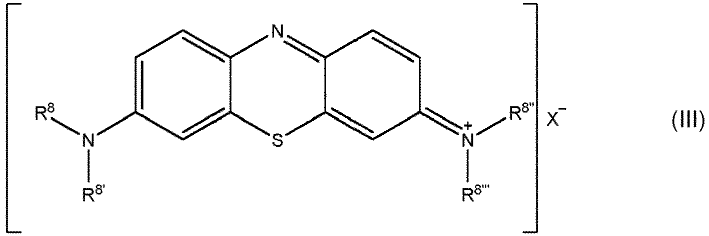
wobei R⁸, R^{8'}, R^{8"} und R^{8‴} unabhängig voneinander entweder für H oder eine C₁₋₄-Alkylgruppe stehen;
oder
wobei R⁸ und R^{8'} und/oder R^{8"} und R^{8‴} zusammen mit N einen fünf- oder sechsgliedrigen Ring bilden;
und X⁻ für ein Anion steht;
mit der Maßgabe, dass
R⁴ von H verschieden ist, wenn R¹ = R² = R³ = CH₃; und
R³ von H verschieden ist, wenn R¹ = R² = R⁴ = CH₃; und
mindestens einer der Reste R¹, R², R³ und R⁴ von H verschieden ist und
mindestens einer der Reste R⁸, R^{8'}, R^{8"} und R^{8‴} von H verschieden ist;
in einer Lösungsmittelmischung von Wasser und mindestens einem C₁₋₈-Alkanol oder mindestens einem C₂₋₄-Alkylendiol;
und unter Verwendung von Licht mit einer Peakwellenlänge (λₘₐₓ) in seinem Spektrum im Bereich zwischen 580 und 780 nm.

2. Verfahren nach Anspruch 1, **dadurch gekennzeichnet, dass** das verwendete Licht eine Peakwellenlänge (λₘₐₓ) in seinem Spektrum im Bereich zwischen 585 und 625 nm aufweist.

3. Verfahren nach Anspruch 1, **dadurch gekennzeichnet, dass** das verwendete Licht eine Peakwellenlänge (λₘₐₓ) in seinem Spektrum im Bereich zwischen 625 und 740 nm aufweist.

4. Verfahren nach einem der vorhergehenden Ansprüche, **dadurch gekennzeichnet, dass** mehr als 80 % des Lichts eine Wellenlänge zwischen 525 und 780 nm aufweisen, vorzugsweise mehr als 80 % des emittierten Lichts eine Wellenlänge zwischen 525 und 700 nm aufweisen und weiter bevorzugt mehr als 65 % des Lichts eine Wellenlänge zwischen 550 und 650 nm aufweisen.

5. Verfahren nach einem der vorhergehenden Ansprüche, **dadurch gekennzeichnet, dass** es sich bei der Lösungsmittelmischung um eine Mischung von Wasser und Methanol und/oder Ethanol und/oder Isopropanol handelt.

6. Verfahren nach einem der vorhergehenden Ansprüche, **dadurch gekennzeichnet, dass** das Volumenverhältnis von Wasser zur Summe von C₁₋₈-Alkanol und C₂₋₄-Alkylendiol im Bereich zwischen 1:10 und 1:1, insbesondere zwischen 1:5 und 1:2, liegt.

7. Verfahren nach einem der vorhergehenden Ansprüche, **dadurch gekennzeichnet, dass** es sich bei der Lichtquelle für das Licht um eine rote oder orangefarbene LED-Lampe handelt.

8. Verfahren nach einem der vorhergehenden Ansprüche, **dadurch gekennzeichnet, dass** es sich bei der Lichtquelle für das Licht um eine weiße LED-Lampe in Kombination mit einem Filter, der Wellenlängen unter 500 nm, insbesondere unter 625 nm, blockiert, handelt.

9. Verfahren nach einem der vorhergehenden Ansprüche, **dadurch gekennzeichnet, dass** R⁸ = R^{8'} = R^{8"} = R^{8‴} = CH₃.

10. Verfahren nach einem der vorhergehenden Ansprüche, **dadurch gekennzeichnet, dass** R¹ und R² und R³ und R⁴ unabhängig voneinander entweder für H oder ein C₁₋₄-Alkyl, vorzugsweise H oder CH₃, stehen, mit der Maßgabe, dass mindestens einer der Substituenten R¹ und R² und R³ und R⁴ nicht für H steht.

11. Verfahren nach einem der vorhergehenden Ansprüche 1-9, **dadurch gekennzeichnet, dass** R⁵ = R⁶ = R⁷ = H ist.

12. Verfahren nach Anspruch 10, **dadurch gekennzeichnet, dass** R² = H und dass R¹ = H oder CH₃.

13. Verfahren nach einem der vorhergehenden Ansprüche, **dadurch gekennzeichnet, dass** X⁻ für ein Halogenid, insbesondere Chlorid, steht.

14. Verfahren nach einem der vorhergehenden Ansprüche, **dadurch gekennzeichnet, dass** die Konzentration der Verbindung der Formel (II) zu Beginn der Photooxidation im Bereich zwischen 0,002 bis 2,0 mol/l, vorzugsweise 0,01 bis 0,2 mol/l, liegt.

15. Verfahren nach einem der vorhergehenden Ansprüche, **dadurch gekennzeichnet, dass** das Verhältnis von Verbindung der Formel (III) zu Verbindung der Formel (II) im Bereich zwischen 0,005 und 20 Mol-%, vorzugsweise zwischen 0,05 und 20 Mol-%, noch weiter bevorzugt zwischen 0,2 und 10 Mol-%, liegt.

16. Verfahren nach einem der vorhergehenden Ansprüche, **dadurch gekennzeichnet, dass** die Photooxidation in einem Strömungsreaktor, insbesondere in einem Spiralströmungsreaktor, durchgeführt wird.

## Revendications

1. Procédé de fabrication d'un composé de la formule (I) à partir d'un composé de la formule (II) par photooxydation 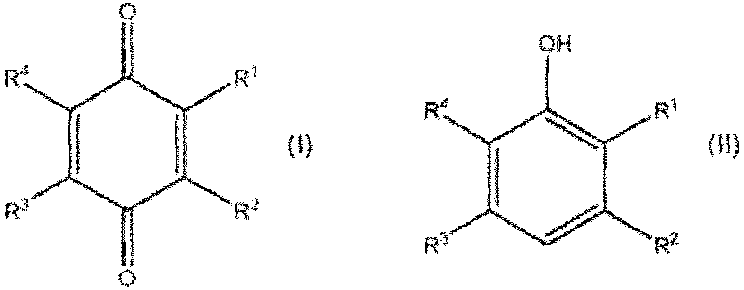
R¹ et R² représentant indépendamment l'un de l'autre soit H, soit un groupe alkyle en C₁₋₄, soit un groupe alkyle halogéné en C₁₋₄, un groupe OR⁹, soit un groupe -CH₂-OR⁹ ;
et R³ et R⁴
soit représentent indépendamment l'un de l'autre H ou un groupe alkyle en C₁₋₄ ou un groupe alkyle halogéné en C₁₋₄ ou un groupe OR⁹ ou un groupe - CH₂-OR⁹, particulièrement H ou CH₃ ;
soit forment ensemble un groupe de la formule 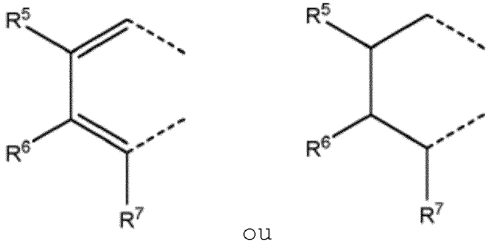
R⁵ et R⁶ et R⁷ représentant indépendamment les uns des autres H ou OH ou un groupe alkyle en C₁₋₄ ou un groupe OR⁹ ;
à la condition qu'au moins deux des radicaux R⁵ et R⁶ et R⁷ soient différents de OH ;
la ligne en pointillés représentant la liaison par laquelle le substituants de la formule est lié au reste du composé de formule (I) ou de formule (II) ;
R⁹ étant un groupe alkyle en C₁₋₄, particulièrement CH₃,
en utilisant de l'oxygène et un agent photosensibilisant de la formule (III) 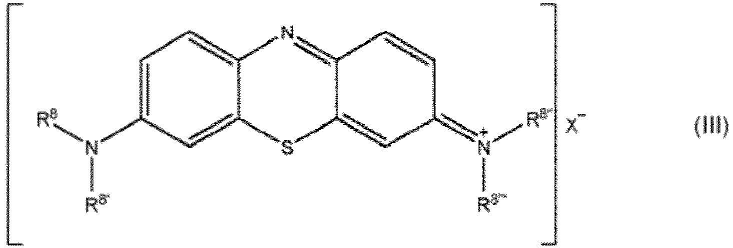
R⁸, R^{8'}, R^{8"} et R^{8‴} représentant indépendamment les uns des autres soit un H, soit un groupe alkyle en C₁₋₄ ;
ou
R⁸ et R^{8'} et/ou R^{8"} et R^{8‴} formant ensemble avec N un cycle à cinq ou six chaînons ;
et X⁻ représentant un anion ;
à la condition que
R⁴ soit différent de H si **R¹=R²=R³ =CH₃;** et
R³ soit différent de H si **R¹=R²=R⁴=CH₃,** et
au moins l'un des radicaux R¹, R², R³ et R⁴ soit différent de H et
au moins l'un des radicaux R⁸, R^{8'}, R^{8"} et R^{8‴} soit différent de H ;
dans un mélange de solvants d'eau et d'au moins un alcanol en C₁₋₈ ou d'au moins un alkylène diol en C₂₋₄ ;
et en utilisant de la lumière qui possède une longueur d'onde maximale (λₘₐₓ) dans son spectre dans la plage comprise entre 580 et 780 nm.

2. Procédé selon la revendication 1 **caractérisé en ce que** la lumière utilisée possède une longueur d'onde maximale (λₘₐₓ) dans son spectre dans la plage comprise entre 585 et 625 nm.

3. Procédé selon la revendication 1 **caractérisé en ce que** la lumière utilisée possède une longueur d'onde maximale (λₘₐₓ) dans son spectre dans la plage comprise entre 625 et 740 nm.

4. Procédé selon l'une quelconque des revendications précédentes **caractérisé en ce que** plus de 80 % de la lumière possède une longueur d'onde comprise entre 525 et 780 nm, préférablement plus de 80 % de la lumière émise possède une longueur d'onde comprise entre 525 et 700 nm, plus préférablement plus de 65 % de la lumière possède une longueur d'onde comprise entre 550 et 650 nm.

5. Procédé selon l'une quelconque des revendications précédentes **caractérisé en ce que** le mélange de solvants est un mélange d'eau et de méthanol et/ou d'éthanol et/ou d'isopropanol.

6. Procédé selon l'une quelconque des revendications précédentes **caractérisé en ce que** le rapport volumique d'eau sur la somme d'alcanol en C₁₋₈ et d'alkylène diol en C₂₋₄ est dans la plage comprise entre 1 : 10 et 1 : 1, particulièrement entre 1 : 5 et 1 : 2.

7. Procédé selon l'une quelconque des revendications précédentes **caractérisé en ce que** la source de lumière pour la lumière est une lampe LED rouge ou orange.

8. Procédé selon l'une quelconque des revendications précédentes **caractérisé en ce que** la source de lumière pour la lumière est une lampe LED blanche en combinaison avec un filtre bloquant des longueurs d'onde en dessous de 500 nm, particulièrement en dessous de 625 nm.

9. Procédé selon l'une quelconque des revendications précédentes **caractérisé en ce que**
**R⁸=R^{8'}=R^{8"}=R^{8‴}= CH₃.**

10. Procédé selon l'une quelconque des revendications précédentes **caractérisé en ce que** R¹ et R² et R³ et R⁴ sont indépendamment les uns des autres soit H, soit un alkyle en C₁₋₄, préférablement H ou CH₃, à la condition qu'au moins l'un des substituants R¹ et R² et R³ et R⁴ ne représente pas H.

11. Procédé selon l'une quelconque des revendications précédentes 1 à 9 **caractérisé en ce que R⁵=R⁶=R⁷ =H,**

12. Procédé selon la revendication 10, **caractérisé en ce que** R² = H et **en ce que** R¹ = H ou CH₃.

13. Procédé selon l'une quelconque des revendications précédentes **caractérisé en ce que** X⁻ représente un halogénure, particulièrement chlorure.

14. Procédé selon l'une quelconque des revendications précédentes **caractérisé en ce que** la concentration du composé de formule (II) est dans la plage comprise entre 0,002 à 2,0 mole/l, préférablement de 0,01 à 0,2 mole/l au début de la photooxydation.

15. Procédé selon l'une quelconque des revendications précédentes **caractérisé en ce que** le rapport du composé de formule (III) sur le composé de formule (II) est dans la plage comprise entre 0,005 et 20 % en moles, préférablement entre 0,05 et 20 % en moles, plus préférablement entre 0,2 et 10 % en moles.

16. Procédé selon l'une quelconque des revendications précédentes **caractérisé en ce que** la photooxydation est réalisée dans un réacteur à écoulement, particulièrement dans un réacteur à écoulement en spirale.
